# EUROPEAN PATENT APPLICATION

(11) **EP 0 732 086 A1**
(43) Date of publication of application: **18.09.1996**
(21) Application number: 96200704.3
(22) Date of filing: 13.03.1996
(51) Int. Cl.: A61F 2/06, A61B 17/36

(54) **Catheter with light conductor**

(30) Priority: 13.03.1995 NL 9500493
(71) Applicant: CORDIS EUROPA N.V., NL-9301 LJ Roden (NL)
(72) Inventor: Ligtenberg, Hendrikus Cornelis Geert, 9351 PX Leek (NL); Haan, Marcel Gerhard, 9312 PE Nietap (NL); Leone, James Ernest, Miami, Florida 33156 (NL)
(74) Representative: 't Jong, Bastiaan Jacobus

(57) **Abstract**

The invention relates to a catheter comprising a tube-like basic body with a distal and a proximal end and a balloon member arranged at the distal end surrounding an end-section of the basic body. At least one light conductor extends from the proximal to the distal end and has, close the distal end, a light-emitting end-section situated inside the balloon member. The light-emitting end-section is attached to the basic body.

## Description

The invention relates to a catheter comprising in the usual manner a tube-like basic body with a distal and a proximal end and to the distal end of which a balloon member has been arranged.

The balloon member surrounds an end-section of the basic body and is supported by it.

With the balloon catheter according to the invention a light conductor extends form the proximal end to the distal end. This light conductor comprises a light-emitting end-section which is situated inside the balloon member. The light-emitting end-section has been attached to the basic body inside the balloon member, in order to secure it inside the balloon.

At the proximal end of the catheter the light conductor has a light-absorbing end, into which light can be admitted which is emitted at the light-emitting end-section.

This balloon catheter according to the invention is particularly suitable for fitting a stent, which has been made of a material which cures due to the action of light, in particular UV-light, inside the body of a patient. Prior to introduction this stent is arranged in compressed state around the non-expanded balloon member. In this state the distal end of the catheter is inserted into the patient, after which the balloon and consequently the stent are manoeuvred into the correct position inside the bloodstream of the patient. By subsequently causing the balloon member to expand by supplying gas or liquid under pressure to it, the stent will be expanded. By next exposing the stent thus introduced to light emitted by the light-emitting end-section, the material of which the stent has been made will cure and the latter will subsequently remain behind in the cured, expanded state inside the body of the patient.

It is important that the light-emitting end-section will be positioned accurately inside the balloon member, in order to achieve an accurate and correct exposure of the curing material.

According to a preferred embodiment, the basic body of the catheter comprises an outer tube-like member and received in a lumen thereof an inner tube-like member and is the light-emitting end-section of the light conductor fixed to the inner tube-like member. Together with the inner tube-like member the light conductor can extend through the lumen of the outer tube-like member.

A suitable other embodiment is characterised in claim 4. The light-emitting end-section can be fixed accurately inside the balloon. By removing at least a section of the outer wall of the catheter bounding the lumen, a kind of bed is obtained which can contribute to keeping the stent in place on introduction.

The light conductor is preferably fixed to the basic body by glueing.

In order to ensure that all round the circumference of the balloon a uniform exposure is achieved, the measure as set out in claim 6 is preferably employed. The light emitted by the light conductor is partly reflected inside the balloon. As a result also that side of the balloon which is situated in the shade cast by the basic body will be exposed properly.

The invention will be explained in greater detail in the following description with reference to the attached drawings.
- Figure 1: shows partly schematically a balloon catheter according to a first embodiment of the invention.
- Figure 2: illustrates the distal end of a balloon catheter according to another embodiment of the invention.
- Figure 3: shows a cross-section along the line III-III of figure 2.

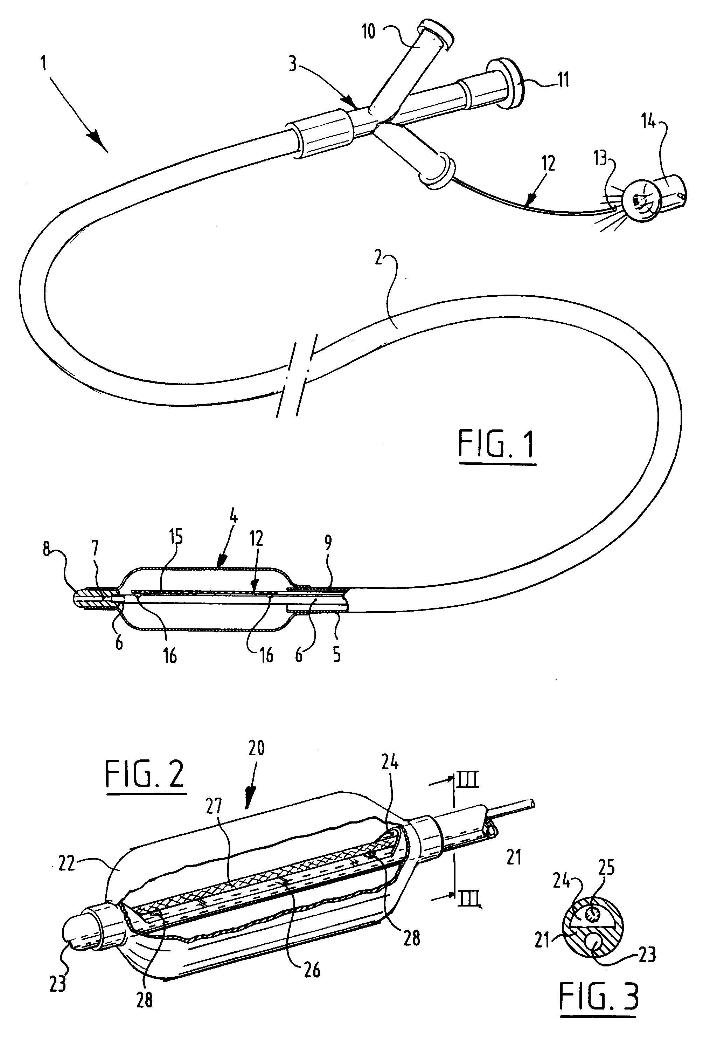

The catheter 1 of figure 1 comprises a tube-like basic body 2 with at a proximal end, that is to say the end which remains outside the body of a patient during treatment, a connecting member 3.

At the opposite, distal end a balloon member 4 has been arranged, which surrounds an end-section of the basic body 2.

The basic body is composed of an outer tube-like body 5 and an inner tube-like body 6 received in a lumen 9 thereof. The inner tube-like body also comprises a lumen 7, through which a guide wire can extend in the usual manner.

At its tip, the inner tube-like body 6 is connected with a catheter-end-section 8, inside of which the guide wire channel 7 is continuated.

The balloon member 4 is connected with its relatively distal end to the catheter-end-section 8 and with its relatively proximal end to the end of the outer tube-like body 5.

Together with the inner tube-like body 6 a light conductor 12 extends through the lumen 9 of the outer tube-like body 5. This light conductor 12 extends from the proximal end to the distal end, so that it comprises a section 15, which is situated inside the balloon member 4 in an uncovered state.

The proximal end-section of the light conductor 12 comprises a light-absorbing end 13 through which, by means of a schematically indicated source of light 14, light can be admitted into the light conductor 12. This light is transmitted inside the light conductor 12 to the light-emitting end-section 15. This light-emitting end-section 15 has preferably been treated in such a way, for instance by grinding, that the admitted light is emitted radially along the entire end-section 15. The end-section 15 is connected with the basic body 2 and in particular with its inner tube-like body 6, by means of glued joints 16.

The balloon member 4 has preferably been made in such a way that the light emitted by the light-emitting end-section 15 is partly reflected and partly transmitted. As a result of the partial reflection, the entire circumference of the inside of the balloon member 4 will be exposed uniformly, also that section which is situated in the direct shade cast by the basic body. This is the bottom section in figure 1. A uniform exposure of the entire circumference of the balloon 4 is necessary in order to effect uniform exposure of the stent to be cured, and with that uniform curing of this stent.

The balloon member 4 is expanded by supplying liquid or gas under pressure to it. For this purpose the remaining cross-section of the lumen 9 is used, which is connected to the connection 10 of the connecting member 3. The connecting member 11 is connected with the guide wire channel 7. This connection 11 comprises a seal so that the guide wire can be advanced in a sealed manner.

Of another embodiment of the catheter, as indicated with the reference number 20, only the distal end-section has been illustrated in fig. 2. The catheter 20 comprises a basic body 21 which, as can be seen more clearly in figure 3, comprises two lumens 23,24. The lumen 23 extends to the very distal end and serves for receiving a guide wire.

A light conductor 25 extends through the lumen 24.

As can be seen in figure 2, the outer wall of the end-section of the basic body 21 extending inside the balloon member 22 and bounding the lumen 24 has partially been removed, so that a "bed" 26 is formed on which the light-emitting end-section 27 of the light conductor 25 has been fixed. Also in this case glue 28 is used for the purpose of fixing.

Also the light-emitting end-section 27 has been treated in such a way that a radial emission of light is effected in order to obtain a uniform exposure along the entire length.

The light conductor can be made of a light-conducting monofilament or a fibre bundle. Also a combination can be employed. The light-emitting end-section 27 can for instance consist of one piece, which has been connected in a light- transmitting manner with a fibre bundle extending from the proximal end.

## Claims

1. Catheter comprising a tube-like basic body with a distal and a proximal end, and a balloon member arranged at the distal end surrounding an end-section of the basic body, a light conductor extending from the proximal to the distal end, and having close to the distal end a light-emitting end-section situated inside the balloon member, said light-emitting end-section being fixed to the basic body, wherein the light conductor extends through a lumen of the basic body and wherein an outer wall of the end-section of the basic body extending into the balloon member and bounding the lumen has at least partially been removed so as to expose said end-section of the light conductor.

2. Catheter as claimed in claim 1, wherein the basic body comprises an outer tube-like body and an inner tube-like body received in a lumen thereof and the light-emitting end-section of the light conductor is fixed to the inner tube-like body.

3. Catheter as claimed in claim 2, wherein the light conductor extends, together with the inner tube-like body, through the lumen of the outer tube-like body.

4. Catheter as claimed in one of the previous claims, wherein the light conductor has been fixed by means of glueing.

5. Catheter as claimed in one of the previous claims, wherein the balloon member has been made in such a way that the light emitted by the light-emitting end-section is partly reflected and partly transmitted.
